# EUROPEAN PATENT APPLICATION

(11) **EP 1 316 313 A2**
(43) Date of publication of application: **04.06.2003**
(21) Application number: 02026909.8
(22) Date of filing: 03.12.2002
(51) Int. Cl.: A61K 38/49

(54) **Composition for the treatment of ischemic stroke containing zonampanel and a tissue plasminogen activator**

(30) Priority: 03.12.2001 US 334556 P; 06.03.2002 US 361724 P
(71) Applicant: YAMANOUCHI PHARMACEUTICAL CO. LTD., Tokyo 103-8411 (JP)
(72) Inventor: Suzuki, Masanaori, Yamanouchi Pharma. Co., Ltd., Tsukuba-shi, Ibaraki 305-8585 (JP); Sasamata, Masao, Yamanouchi Pharma. Co., Ltd., Tsukuba-shi, Ibaraki 305-8585 (JP); Sumii, Toshihisa, Osakasayama-shi, Osaka 589-0021 (JP); Lo, Eng H., Newton, Massachusetts 02466 (US)
(74) Representative: HOFFMANN - EITLE

(57) **Abstract**

The present invention relates to a combination of [7-(1H-imidazol-1-yl)-6-nitro-2, 3-dioxo-3, 4-dihydroquinoxalin-1(2H)-yl]acetic acid or a salt or hydrate thereof or, preferably, zonampanel together with a tissue plasminogen activator for the therapy of ischemic stroke or for the improvement of neurological symptom accompanied by cerebral infarction, to a method of administration thereof and to a method of therapy therefor. The combination of the present invention showed better effect of reducing the infarct volume than administration of a single component.
Therefore, the combination of the present invention is useful as a therapy for ischemic stroke.

## Description

### Technical Field

The present invention relates to a combination of [7-(1H-imidazol-1-yl)-6-nitro-2,3-dioxo-3,4-dihydroquinoxalin-1(2H)-yl]acetic acid(hereinafter referred to as zonampanel) or a salt or hydrate thereof or, preferably, zonampanel monohydrate (hereinafter referred to as compound A) together with a tissue plasminogen activator for the therapy of ischemic stroke or for the improvement of neurological symptom accompanied by cerebral infarction, to a method of therapy using these active ingredients and to a administration method of zonampanel or a salt or hydrate thereof.

### Background Art

Cerebral infarction is an organic cerebral pathology in which cerebral blood vessel is obstructed by arteriosclerosis of local blood vessel or by thrombus carried thereto and the blood flow thereafter is stopped whereby energy of cerebral cells is exhausted and, in final, cell death occurs forming infarct foci.

Cerebral infarction is classified into thrombotic (cerebral thrombus), embolic (cerebral embolism) and hemodynamic ones depending upon the origin of thrombus. In cerebral thrombus, arteriosclerosis is formed in artery in brain and adhesion and aggregation of platelets are resulted on its diseased site to form thrombus. In the patients suffering from cerebral thrombus, there are many cases having underlying diseases (hypertension, diabetes mellitus, hyperlipidemia, etc.) accompanied by arteriosclerosis.

In cerebral embolism, thrombus formed in blood vessel except in brain is carried to brain by blood flow and obstructs the cerebral blood vessel. In the patients suffering from cerebral embolism, there are many cases having cardiac disease and arteriosclerosis of cervical artery.

In hemodynamic cerebral infarction, there occur reduction in blood pressure and cardiac output, significant bradycardia and dehydration at the site where strong stenosis or infarction for supplying of artery blood near and to brain happened previously whereby the onset takes place to form infarct foci in the boundary region or the terminal region. (*Nokekkan Shogai - Sono Rinshoteki Apurochi,* pages 14-17, edited by Takenori Yamaguchi and Makoto Takagi, Kabushiki Kaisha Igaku Shoin, Tokyo, 1994).

On the other hand, according to the classification in view of clinical disease type, it is classified into atherothrombic cerebral infarction, cardiogenic cerebral embolism, lacunar infarction and others *(Stroke,* 21, 637-376, 1990).

Atherothrombic cerebral infarction occurs on the basis of a strong atherosclerosis of artery of the brain and the neck and, in view of mechanism of onset of the infarction, there are included cerebral infarction occurred by obstruction of thrombus at its controllable region, infarction of peripheral perfusion area occurred via a mechanism of arteriogenic cerebral embolism, watershed infarction due a hemodynamic mechanism, etc.

Cardiogenic cerebral embolism is resulted by such a manner that thrombus formed in the heart is liberated and obstructs the artery of neck or brain according to the same mechanism as in the case of cerebral infarction.

Lacunar infarction is a small lesion generated in a penetrating artery region in a deep area of cerebrum.

From electrophysiological and molecular biological studies, it has been known that glutamic acid receptors are classified into subtypes of NMDA (N-methyl-D-aspartic acid) receptor, AMPA (α-amino-3-hydroxy-5-methyl-4-isoxazole propionate) receptor, kainic acid receptor and glutamic acid receptor of a metabolic type.

Zonampanel or a salt or hydrate thereof (hereinafter referred to as zonampanel or a salt etc thereof.) is a [7-(1H-imidazol-1-yl)-6-nitro-2,3-dioxo-3,4-dihydroquinoxalin-1(2H)-yl]acetic acid which is an AMPA receptor antagonist disclosed in WO 96/10023 and zonampanel monohydrate (YM872, hereinafter referred to as compound A) has a strong and selective AMPA binding activity and a high solubility in water. Zonampanel shows a neuroprotective effect by suppressing an intracellular calcium influx by AMPA (1). Further, zonampanel or a salt etc. thereof shows a neuroprotective action in various cerebral infarction models (2, 3, 4, 5, 6, and 7). Furthermore, in zonampanel or a salt etc. thereof and NBQX (2,3-dihydroxy-6-nitro-7-sulfamoyl-benzole (F) quinoxaline) which is an AMPA receptor antagonist, a neuroprotective action is noted even when administered after several hours from the onset of infarction (2, 3, 14 and 15).

From the above, zonampanel or a salt etc. thereof is expected to be a medicament which is able to suppress the occurrence of sequelae by its administration in the acute phase of ischemic cerebrovascular disorders although the clinical effect of other AMPA receptors is not clarified.

On the other hand, alteplase which is a native recombinant human tissue plasminogen activator was approved in the United States in 1996 as a therapeutic agent for administration in the acute phase of ischemic stroke (within 3 hours after the onset) and, since then, alteplase has been an only therapeutic agent for the therapy of acute phase of ischemic stroke.

The action of alteplase for reducing the infarct foci is believed to be due to a recovery of cerebral blood flow in an early phase by its thrombolytic action (9). However, it has no effect of suppressing the nervous cell death of cerebral cells which are once exposed to ischemia.

It is therefore expected that, in the therapy of the acute phase of ischemic stroke, zonampanel or a salt etc. thereof which is an AMPA receptor antagonist is jointly administered in addition to a thrombolytic treatment by a tissue plasminogen activator whereby the therapeutic effect to ischemic stroke is further enhanced.

Incidentally, in the level of the experiments using animals, the effects by a combined use of alteplase with NBQX which is an AMPA receptor antagonist have been reported (12 and 13) but its effects of improving the neurological symptoms are the same (12) or rather lower (13) as compared with a group to which alteplase is solely administered. On the other hand, there are many cases where the patients of cerebral stroke in an acute phase have hypertension and, in the acute phase of ischemic stroke, a hypotensive therapy is said to be principally incompatible *(Medicina,* 37(7), 1135-1138, 2000). Accordingly, even in ischemic stroke accompanied with hypertension, the fact that a combination administration of a tissue plasminogen activator with zonampanel has a neuroprotective action is useful in the therapy in the acute phase of ischemic stroke.

It is expected that AMPA receptor antagonists can be a medicament for occurrence of sequelae by administration during an acute phase of ischemic cerebrovascular disorders although their effect to cerebral infarction models using spontaneously hypertensive rats have not been known.

On the other hand, the effect of pamiteplase which is a modified tissue plasminogen activator to ischemic stroke model having normal blood pressure has been reported *(Neuroreport,* 12(3), 615-618, 2001) although its effect to cerebral infarction models using spontaneously hypertensive rats used in the present invention has not been known. Further, although the effect of alteplase to cerebral infarction models using spontaneously hypertensive rats has been investigated, the effect is insufficient *(Stroke,* 32(6), 1336-1340, 2001; *Journal of Cerebral Blood Flow & Metabolism,* 20(3), 452-457, 2000; *Neurology,* 52(7), 1381-1384, 1999; and *Chemical & Pharmaceutical Bulletin,* 39(12), 3327-3330, 1991).

### Disclosure of the Invention

The present inventors have investigated the therapeutic effect for ischemic stroke using various cerebral infarction models by the combination use of zonampanel or a salt etc. thereof during the administration of a tissue plasminogen activator and, unexpectedly, found that the combination use of zonampanel or a salt etc. thereof has a significant effect of reducing the infarct volume and improving the neurological symptoms as described later whereupon the present invention has been achieved.

Thus, the present invention relates to:
a method for the treatment of ischemic stroke, which comprises administering to a patient a) a therapeutically effective amount of a tissue plasminogen activator as the first component and, (b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component;
preferably, the above method wherein the second component is administered together with or with a time interval from the administration of the first component;
a method for the improvement of insufficiency accompanied by cerebral infarction, which comprises administering to a patient a) a therapeutically effective amount of a tissue plasminogen activator as the first component and (b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component;
preferably, the method wherein the above insufficiency is a neurological symptoms;
more preferably, the above method wherein the first component is a tissue plasminogen activator selected from the group consisting of alteplase and pamiteplase;
a method for the suppression of progress of cerebral infarction, which comprises administering to a patient a) a therapeutically effective amount of a tissue plasminogen activator as the first component and (b) [7-(1H-imidazol-1-yl)-6-nitro-2,3-dioxo-3,4-dihydroquinoxalin-1(2H)-yl]acetic acid or a salt or hydrate thereof as the second component;
a combination for the treatment of ischemic stroke, which comprises a) a tissue plasminogen activator as the first component with (b) [7-(1H-imidazol-1-yl)-6-nitro-2,3-dioxo-3,4-dihydroquinoxalin-1(2H)-yl]acetic acid or a salt or hydrate thereof as the second component;
preferably, a combination for the treatment of ischemic stroke, which comprises a) a tissue plasminogen active as the first component with b) [7-(1H-imidazol-1-yl)-6-nitro-2,3-dioxo-3,4-dihydroquinoxalin-1(2H)-yl]acetic acid or a salt or hydrate thereof as the second component and, in the combination, the first and second components are intended for different uses in the same or the successive phase or in the apart phases in terms of time;
more preferably, the above combination wherein the first component is a tissue plasminogen activator selected from the group consisting of alteplase and pamiteplase;
the use of a combination of a tissue plasminogen activator with [7-(1H-imidazol-1-yl)-6-nitro-2,3-dioxo-3,4-dihydroquinoxalin-1(2H)-yl]acetic acid or a salt or hydrate thereof for the manufacture of a medicament for the treatment of ischemic stroke; and,
preferably, the above use wherein the tissue plasminogen activator is selected from the group consisting of alteplase and pamiteplase.

Furthermore, the present invention relates to:
a pharmaceutical composition for the improvement of function insufficiency accompanied by cerebral infarction, which comprises [7-(1H-imidazol-1-yl)-6-nitro-2,3-dioxo-3,4-dihydroquinoxalin-1(2H)-yl]acetic acid or a salt or hydrate thereof in a therapeutically effective amount when administered to a patient suffered from ischemic stroke and has administered a therapeutically effective amount of a tissue plasminogen activator;
a pharmaceutical composition as above, wherein the composition is begun administering to the patient during the period of a tissue plasminogen activator administration;
the use of [7-(1H-imidazol-1-yl)-6-nitro-2,3-dioxo-3,4-dihydroquinoxalin-1(2H)-yl]acetic acid or a salt or hydrate thereof for the manufacture of a medicament for the improvement of function insufficiency accompanied by cerebral infarction, which is administered to a patient to which a therapeutically effective amount of a tissue plasminogen activator has been administered;
the use of said compound or a salt or hydrate thereof for the manufacture of a medicament as above, wherein the medicament is begun administering to the patient during the period of a tissue plasminogen activator administration;
a pharmaceutical composition as above, wherein the composition is begun administering to the patient during the period between an onset of ischemic stroke and four hours after thereof;
the use of said compound or a salt thereof for the manufacture of a medicament as above, wherein the medicament is begun administering to the patient during the period between an onset of ischemic stroke and four hours after thereof;
a pharmaceutical composition comprising [7-(1H-imidazol-1-yl)-6-nitro-2,3-dioxo-3,4-dihydroquinoxalin-1(2H)-yl]acetic acid or a salt or hydrate thereof in a therapeutically effective amount for potentiating the reducing effect of cerebral infarction volume by treatment a tissue plasminogen activator to the patient during the period between an onset of ischemic stroke and four hours after thereof; and
the use of [7-(1H-imidazol-1-yl)-6-nitro-2,3-dioxo-3,4-dihydroquinoxalin-1(2H)-yl]acetic acid or a salt or hydrate thereof for the manufacture of a medicament for potentiating the reducing effect of cerebral infarction volume by treatment a tissue plasminogen activator, wherein the medicament is administered to a patient during the period between an onset of ischemic stroke and four hours after thereof.

Furthermore, the present invention relates to:
a method for improving function insufficiency accompanied by cerebral infarction, which comprises administering a therapeutically effective amount of [7-(1H-imidazol-1-yl)-6-nitro-2,3-dioxo-3,4-dihydroquinoxalin-1(2H)-yl]acetic acid or a salt or hydrate thereof to a patient suffered from ischemic stroke and has administered a therapeutically effective amount of a tissue plasminogen activator;
the above method wherein administration to the patient starts during the period of a tissue plasminogen activator administration.
the above method wherein the administration to the patient starts during the period between an onset of ischemic stroke and four hours after thereof; and
a method for potentiating the reducing effect of cerebral infarction volume by treatment a tissue plasminogen activator, which comprises administering a therapeutically effective amount of [7-(1H-imidazol-1-yl)-6-nitro-2,3-dioxo-3,4-dihydroquinoxalin-1(2H)-yl]acetic acid or a salt or hydrate thereof to a patient during the period between an onset of ischemic stroke and four hours after thereof.

The order of administrations of the first component and the second component is not particularly limited and the case wherein the second component is administered earlier is included in the present invention.

The second component is preferably administered concurrently with the administration of the first component or during the administration of the first component.

The term "in the apart phases in terms of time" in the present invention means that each of the components is administered with a time difference to a patient who requires the therapy for ischemic stroke.

The term "are intended for different uses" used herein means that the administration of components may not be concurrent.

The term "a patient" here means a patient who is suffered form ischemic stroke, and is needed to treat or has been treated with tissue plasminogen activator.

Suppression of the progress of cerebral infarction means an effect that expansion of infarct nidus with a lapse of time after the onset of ischemic event is suppressed as compared with the untreated case.

Clinically, it means that case where the degree of observation of infarct nidus by means of CT for diagnosis carried out immediately after the ischemic event is light as compared with the state and the degree of ischemia.

The term "CT" herein is used to generally represents the "computed tomography" and includes X-ray CT, MRI, etc.

Method for the manufacture of the compounds used in the present invention is disclosed in the patents wherein such compounds are disclosed. Method for the synthesis of [7-(1H-imidazol-1-yl)-6-nitro-2,3-dioxo-3,4-dihydroquinoxalin-1(2H)-yl]acetic acid or a salt or hydrate thereof is disclosed in WO 96/10023.

[7-(1H-imidazol-1-yl)-6-nitro-2,3-dioxo-3,4-dihydroquinoxalin-1(2H)-yl]acetic acid forms a salt with acid or base. Preferably, it is a pharmaceutically acceptable salt.

Examples of the salt with acid are acid addition salts with inorganic acid such as hydrochloric acid, hydrobromic acid, hydroiodic acid, sulfuric acid, nitric acid, phosphoric acid and other mineral acid and organic acid such as formic acid, acetic acid, propionic acid, oxalic acid, malonic acid, succinic acid, fumaric acid, maleic acid, lactic acid, malic acid, tartaric acid, carbonic acid, picric acid, methanesulfonic acid, ethanesulfonic acid and glutamic acid. Examples of the salt with base are the salts with inorganic base such as sodium, potassium, magnesium, calcium and aluminum; organic base such as methylamine, ethylamine and ethanolamine; basic amino acid such as lysine, arginine and ornithine; and ammonium salt. It is also possible to form a hydrate, a solvate with ethanol, etc. and crystal polymorph. [7-(1H-imidazol-1-yl)-6-nitro-2,3-dioxo-3,4-dihydroquinoxalin-1(2H)-yl]acetic acid forms a hydrate. A preferred one is [7-(1H-imidazol-1-yl)-6-nitro-2,3-dioxo-3,4-dihydroquinoxalin-1(2H)-yl]acetic acid monohydrate. Furthermore zonampanel can form solvate with ethanol and so on. Polymorphism might be existance in a salt, hydrate or solvate of zonampanel.

A tissue plasminogen activator (tPA) has a high affinity to fibrin, activates the plasminogen which is bonded to fibrin and dissolves thrombus quite efficiently. The tissue plasminogen activator includes that of a native type and that of a modified type where, as one of the means for supplementing the disadvantage of the tissue plasminogen activator of native type, modification is carried by a genetic engineering means to extend the half life in blood.

Examples of the tPA preparation of a native type are alteplase, tisokinase, liteplase and nateplase.

Examples of the tPA preparation of a modified type are pamiteplase, monteplase, lanoteplase, tenecteplase, amediplase, K2P, CGP-42935 and LY-210825.

Preferred examples are alteplase and pamiteplase.

Alteplase is a generic name of a recombinant human tissue plasminogen activator (rt-PA) of a native type which is a glycoprotein comprising 527 amino acids developed by Genentech and is sold by and available from Genentech as Activase (registered trade mark).

Pamiteplase is a tissue plasminogen activator of a modified type comprising 445 amino acid residues where, in an amino acid sequence of a human tissue plasminogen activator, 92nd to 173rd amino acid residues are deficient and the 275th arginine residue is substituted with glutamic acid residue (refer to the specification of Japanese Patent No. 2,534,332).

A combination of a tissue plasminogen activator with zonampanel or a salt etc. thereof used in the present invention can be administered either orally or parenterally as a pharmaceutical composition where each of the components is separately or simultaneously mixed with physiologically acceptable carrier, excipient, binder, diluent, etc. When each effective component is separately made into a pharmaceutical preparation, such separate preparations may be mixed using a diluent, etc. in actual use and administered or may be administered to the same object separately, simultaneously or with a time difference.

Preferably, zonampanel or a salt etc. thereof is administered together with the administration of a tissue plasminogen activator or within one hour from the initiation of administration of a tissue plasminogen activator.

Cerebral infarction is a state, as defined already, that ischemic area is locally generated in brain by obstruction or perfusion pressure decrease of cerebral blood vessel whereby an irreversible necrosis of neurons occurs.

It is preferably an acute ischemic stroke phase within 48 hours of stroke onset, more preferably within 24 hours, still more preferably within 6 hours and, most preferably, ischemic stroke within 3 hours of stroke onset.

The term "onset" is defined as the time that which the patient was last seen in a normal state, or bedtime for unwitnessed strokes occurring during the night.

In the case of an administration of tissue plasminogen activator is started at 3 hours after stroke onset, during the administration, i.e. ischemic stroke within 4 hours of stroke onset, is intended to ishcemic stroke of the present invention.

Due to the cause of thrombus, cerebral infarction is classified into cerebral thrombus and cerebral embolism and the present invention is useful for the therapy of cerebral thrombus and cerebral embolism. Preferably, it is cerebral infarction without bleeding in brain.

Therapy of ischemic stroke means an effect of preventing the progress of infarct foci in an acute phase of ischemic stroke, an effect of improving the dysfunction or the subjective symptom accompanied by cerebral infarction and/or an effect of preventing the occurrence of psychiatric symptom and convulsion onset during a chronic phase. It further includes prevention of recurrence of the onset of ischemic stroke.

In addition, according to the observation by CT prior to the administration, degree of cerebral infarction may be classified depending upon infarct size, extent of infarct foci (penetrating branch and cortical branch), side of infarct (left, right or both), region of infarct (anterior cerebral artery region, middle cerebral artery region, posterior cerebral artery region, watershed region, brain stem, cerebellum and others) and degree of edema.

Dysfunction accompanied by cerebral infarction means neurological symptoms, movement disorder of daily life, motor paralysis, etc. which occur during the acute phase of ischemic stroke.

Such symptoms may be expressed by the index of degree of disturbance and neural function using modified Rankin scale, Barthel index, NIHSS and Glasgow scale and by a neurological check (evaluation of consciousness level, pupillary symmetry vs. optical reaction, motor ataxia and clonic muscular convulsion, etc.).

To be more specific, clinical symptoms of the following items are observed and expressed in numerals.

### Disturbance of consciousness;

Subjective symptoms (headache, dull headache, dizziness, numbness of extremities, etc.);

Phychiatric syndromes (reaction to being called by name and to greeting, orientation, volition, knowledge, computing ability, voice tone, attitude, spontaneous motion, spontaneous speaking, carefulness, etc.);

Neurological syndromes (aphasia, apraxia, agnosia, dyslalia, dysphagia, muscle strength at the side of motor paralysis, sensory disorder, etc.); and

Movement disorder of daily life (rolling over in bed, keeping the state of sitting, standing up, walking, dressing and undressing oneself, taking the meals, urination/evacuation, etc.).

Neurological symptom means the state where disorder is noted in one or more of the above-mentioned items for neurological syndromes.

Psychiatric symptom at the chronic phase means the psychiatric symptom occurring three weeks and thereafter from the onset of ischemic stroke and its specific examples are lowering of spontaneousness (hypobulia), dysmnesia, anxiety, depressiveness, distemper, psychomotor agitation, delusion, labile affect, aggressive action, mental excitation, ecdemomania, delirium, etc.

Pharmaceutical preparations comprising zonampanel or a salt etc. thereof are manufactured using carrier, filler and other additives which are commonly used for corresponding types of preparations.

Carrier and filler used for pharmaceutical preparations may be solid such as lactose, magnesium, stearic acid, starch and talc and liquid such as gelatin, gum, pectin, acacia, olive oil, cacao butter and ethylene glycol. Other conventionally used carriers and fillers may be used as well.

The combination of the present invention can be administered by various administration modes including mainly by parenteral administration or, to be specific, subcutaneous administration, intramuscular administration, intravenous administration, percutaneous administration, intraspinal administration, epidural administration, intra-articular administration and local administration or by oral administration, if possible.

Injection for parenteral administration includes aseptic aqueous or non-aqueous solutions, suspensions and emulsions. The aqueous solutions and emulsions include distilled water for injection and physiological saline solution. Examples of the non-aqueous solutions and suspensions are propylene glycol, polyethylene glycol, vegetable oil such as olive oil, alcohol such as ethanol, Polysolvate 80 (trade name), etc. Such composition may further contain auxiliary agents such as antiseptic agent, moisturizer, emulsifier, dispersing agent, stabilizer (such as lactose) and solubilizing aid (such as meglumic acid). They may be sterilized by, for example, means of filtration passing through a bacteria-retaining filter, compounding with a sterilizer or irradiation. The combination of the invention may be also made into an aseptic solid composition and dissolved in aseptic water or in an aseptic solvent for injection before use.

Dose of zonampanel or a salt etc. thereof is appropriately determined for each case depending upon symptom, sex and age of the object to be administered, etc. and, usually, it is 100-2000 mg per day or, preferably, approximately 900 mg per day for an adult. It is also possible to administer 100-2000 mg per day to an adult once daily or by dividing into 2 to 4 times a day. In the case of intravenous or continuous intravenous administration, it is possible to be administrated for 1 hour to 24 hours per day.

As mentioned above, the dose may be determined depending upon various conditions. The dose which is less than the above-mentioned range may be used when that is effective.

Preferably, together with the administration of a tissue plasminogen activator simultaneously or within 1 hour from the start of the administration of a tissue plasminogen activator, 1.0 mg/kg/hour of zonampanel monohydrate is administered intravenously for continuous 6 hours and, after that, 0.5 kg/kg/hour is infused for continuous 18 hours.

Dose of the tissue plasminogen activator is appropriately determined for each case by taking type of the medicament used, symptom, age and sex of the object to be administered, etc. into consideration.

In the case of alteplase, it is usually 50-100 mg for an adult (incidentally, 1 mg of alteplase corresponds to 580,000 international units (IU)) or, preferably, 0.9 mg/kg per day. Method for the administration may be that 50-100 mg per day is administered at a time or by dividing into 2 to 4 times a day. In the case of intravenous administration or continuous intravenous administration, administration for 1 hour to 24 hours per day is also possible. Preferably, 10% of 0.9 mg/kg (upper limit being 90 mg per person) is quickly administered (bolus administration) intravenously in 1 to 2 minutes and the remainder is subjected to a continuous intravenous administration for 1 hour.

In the case of pamiteplase, it is usually preferred that from 32,500 IU/kg to 93,000 IU/kg or, preferably, from 32,500 IU/kg to 65,000 IU/kg per day for an adult is subjected to an intravenous bolus administration and an intravenous injection within one minute is preferred. Incidentally, the amount of pamiteplase in an injection solution is about 0.1 mg/ml or more and 1 mg of pamiteplase corresponds to 650,000 IU.

### Brief Description of the Drawings

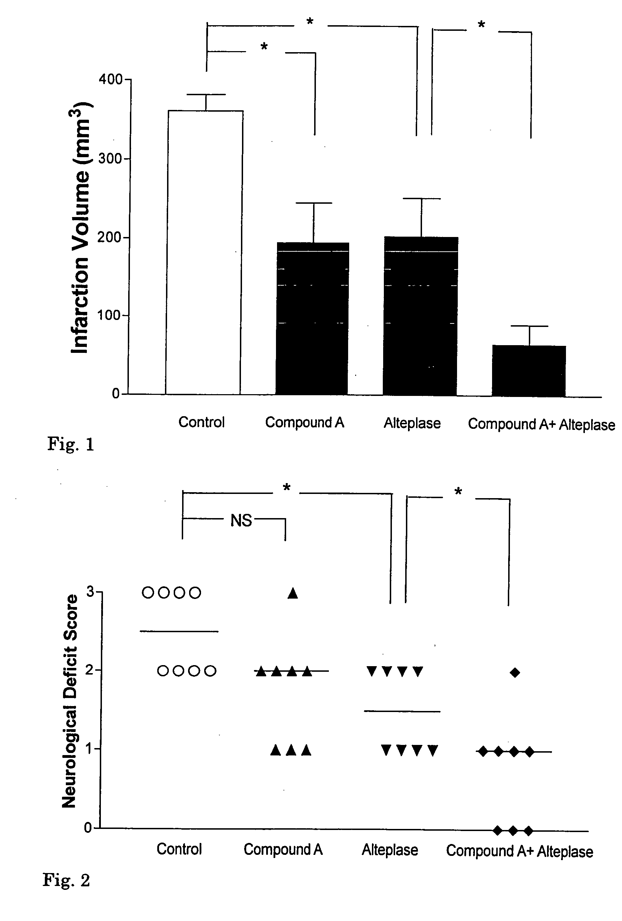

Fig. 1: Effect of a combined use of compound A with alteplase on infarct volume in rat embolic stroke models. Administration of compound A (20 mg/kg/hr; i.v. for 4 hours) and alteplase (10 mg/kg; i.v.; 10% of the total dose was subjected to an intravenous bolus administration while the remainder (90%) was subjected to an intravenous administration continuously over 1 hour) was initiated 2 hours after the injection of blood clot. After 24 hours from the infusion of the blood clot, the brain was excised, sliced and stained with TTC. The data in the drawing show a mean value ± standard error (n = 8). The symbol * means a statistically significant difference from a control group or an alteplase-administered group (* p < 0.05; Dunnett's test or t-test).

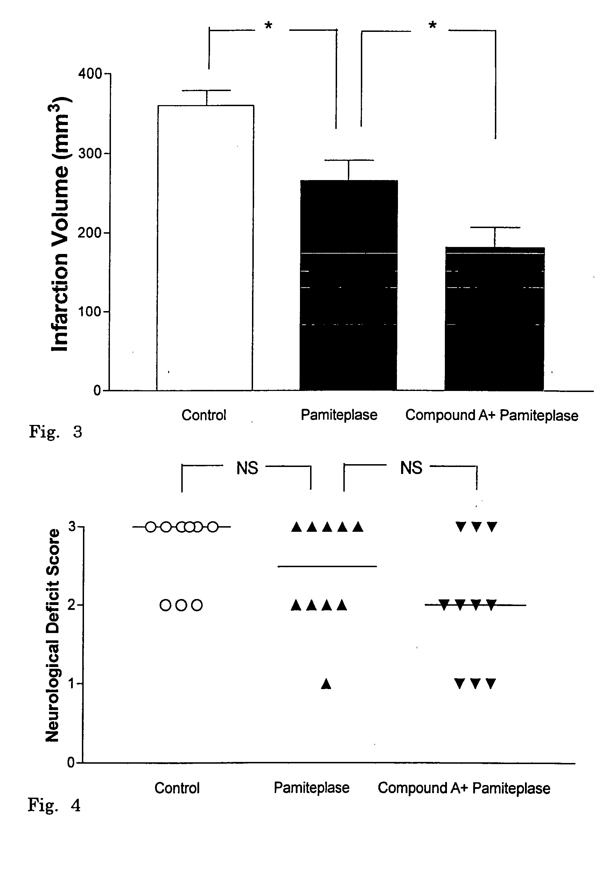

Fig. 2: Effect of a combined use of compound A with alteplase on neurological symptoms in rat embolic stroke models. Administration of zonampanel (20 mg/kg/hr; i.v. for 4 hours) and alteplase (10 mg/kg; i.v.; 10% of the total dose was subjected to an intravenous bolus administration while the remainder (90%) was subjected to an intravenous administration continuously over 1 hour) was initiated 2 hours after the injection of blood clot. After 24 hours from the injection of the blood clot, the neurological symptoms were observed. Data in the drawing show the data derived from each animal (n = 8). The symbol * means a statistically significant difference from a control group or an alteplase-administered group (* p < 0.05; Steel's test or Wilcoxon-test).
Fig. 3: Effect of a combined use of compound A with pamiteplase on infarct volume in rat cerebral embolic models. Administration of compound A (20 mg/kg/hr; i.v. for 4 hours) and pamiteplase (1 mg/kg; i.v.) was initiated 1 hour after the infusion of blood clot. After 24 hours from the injection of the blood clot, the brain was excised, sliced and stained with TTC. The data in the drawing show a mean value ± standard error (n = 10). The symbol * means a statistically significant difference from a control group or a pamiteplase-administered group (* p < 0.05; t-test).
Fig. 4: Effect of a combined use of compound A with pamiteplase on neurological symptoms in rat cerebral embolic models. Administration of compound A (20 mg/kg/hr; i.v. for 4 hours) and pamiteplase (1 mg/kg; i.v.) was initiated 1 hour after the injection of blood clot. After 24 hours from the injection of the blood clot, the neurological symptoms was observed. Data in the drawing mean the data derived from each animal (n = 10). The term NS means that there is no statistically significant difference from a control group or a pamiteplase-administered group (Wilcoxon test).

### Best Mode for Carrying Out the Invention

The present invention will now be illustrated in more detail by way of the following examples although the present invention is not limited those examples only.

### Example 1. Effect by Combined Use of Alteplase with compound A

### <Experimental Methods>

1) Preparation of Embolic Stroke Models
   As to the embolic stroke models, male Sprague-Dawley rats (Charles River Japan) each having a body weight of 280-350 g were used. The method of Kudo, et al. (8) was followed with a partial modification (9, 10) for the preparation of the embolic stroke models. The rats for the collection of blood were anesthetized with 1.0% halothane (Fluothane®; Takeda Chemical Industries) under a spontaneous respiration. A 24-gauge Surflo® needle (Terumo) was secured in the femoral artery and 0.1 mL of arterial blood was taken with a 1-mL syringe for injection (Terumo). The artery blood in the syringe was incubated at 30°C for 2 days to form a blood clot. After that, 0.1 mL of a physiological saline was added to the syringe for injection and passed through a 26-gauge injection needle (Terumo) twice so that the blood clot was crushed.
   Rats for the preparation of cerebral embolic stroke were anesthetized with 1.0% halothane under a spontaneous respiration. Neck of the rats was subjected to a midline incision and external carotid artery, superior thyroid artery, occipital artery and pterygopalatine artery were cauterized with a bipolar coagulator (T-45; Keisei Medical). Cerebral embolism was induced by injecting 0.1 mL of the crushed blood clot into the internal carotid artery from the external carotid artery. During the experiment, body temperature of the animals was maintained approximately at 37°C using a heating pad for small animals (BWT-100; Bio Research Center).
   Evaluation of formation of cerebral embolism by injection of blood clot was carried out using a laser Doppler flowmetry (FloC1; Omegawave) and the cerebral blood flow decreased to a level of 30% or less of the blood clot value immediately after ischemia was taken as a positive evidence of embolism formation. The probe for the blood flow measurement was applied on the top of the forehead. The cerebral blood flow was monitored until 30 minutes after infusion of the blood clot and the blood flow of 30 minutes after ischemia was confirmed to remain 50% or less than the value prior to the injection of the blood clot. After that, a cannula (PE5O) for administration of the medicament was secured in the jugular vein and the animals were awoken.
2) Administration of Medicaments
   With regard to alteplase (Activase (registered trade mark); Genentech; Lot E9503A), the commercially available product was purchased. Both medicaments were started in intravenous injection through a cannula secured in the common jugular vein 2 hours after injection of the blood clot. The dose of alteplase was set at 10 mg/kg and 10% of the total dose was subjected to a bolus administration intravenously while the remainder (90%) was continuously administered over 1 hour intravenously. With regard to zonampanel or a salt etc. thereof, it was continuously administered over 4 hours intravenously at a dose of 20 mg/kg/hour as zonampanel monohydrate (hereinafter referred to as compound A).

### <Items for the Evaluation>

1) Infarct Volume
   The animals were sacrificed about 24 hours after infusion of the blood clot and the brain was excised. The excised brain was sliced in ten sections at 1 mm intervals using a McIwain tissue chopper (Mickle Laboratory Engineering) and stained by dipping for 20 minutes in a 2% TTC (2,3,5-triphenyltetrazolium chloride; Tokyo Kasei) at 37°C. Images of the TTC-stained slices were uploaded into a computer using a digital camera (HC-2500; Fuji Photo-Film) and Phatograb-2500 (Fuji Photo Film) and infarct volume was calculated using Mac Scope (Mitani).
2) Neurological Symptoms
   Neurological symptoms were observed 24 hours after the infusion of the blood clot. Scoring of the neurological symptoms was carried out according to a method of Bederson et al. (11). Criteria of the score are as follows. Thus, score 0: normal and no worsening of the symptom; score: 1: when the animal was taken up by clipping the tail, forelimb was flexed; score 2: weak resistance to the pushing force from the side and forelimb flexion noted while no circling observed; and score 3: the same symptom as in 2 and the circling were noted.
3) Statistical Means
   Result of the infarct volume was given by a mean value ± standard error while result of neurological symptom was given by a median value. With regard to the statistical test of the result of the infarct volume, the evaluation was done by carrying out a Dunnett's test for control group and for each of alteplase-administered group and zonampanel-administered group as compared with the control group and then by carrying out the t-test for the alteplase-administered group and the zonampanel-alteplase-administered group. On the other hand, with regard to the statistical test of the result of neurological symptoms, the evaluation was done by carrying out a Steel's test for control group and for each of alteplase-administered group and zonampanel-administered group as compared with the control group and then by carrying out a Wilcoxon test for the alteplase-administered group and the zonampanel-alteplase-administered group. In any of the tests, the value where p < 0.05 was defined to be statistically significant.

### <Results>

1) Infarct Volume
   As a result of injection of the blood clot, the cerebral blood flow decreased to about 30% or less than the value prior to the infusion and cerebral embolism was formed. The medicament was administered 2 hours after the injection of the blood clot.
   Result of the infarct volume is shown in Fig. 1. The infarct volume of a control group (a group to which a physiological saline was administered) was 361.2 ± 20.9 mm³ (mean value ± standard error). Alteplase (10 mg/kg) and compound A (20 mg/kg/hour for 4 hours) reduced the infarct volumes to 202.7 ± 49.3 mm³ and 194.6 ± 50.6 mm³, respectively. When compound A (20 mg/kg/hour for 4 hours) and alteplase (10 mg/kg) were jointly used, the infarct volume became 64.4 ± 25.3 mm³ and the said volume was smaller than that for the alteplase-administered group.
2) Neurological Symptoms
   Result of the neurological symptoms is shown in Fig. 2. The neurological symptom in the control group worsened to a score of 2.5 (median value). By a sole administration of compound A (20 mg/kg/hour for 4 hours), the neurological symptom became a score of 2 showing the tendency of improvement (p = 0.07). When zonampanel and alteplase were jointly used, the neurological symptom gave a score of 1 and a more statistically significant improvement was noted than the alteplase-administered group.
   With regard to the effect to ischemic stroke by the combined use of alteplase with compound A according to the present invention, the infarct volume was statistically significantly reduced as compared with a sole administration of alteplase and the neurological symptom was statistically significantly improved as well.
   From the above, better effect was confirmed by the combined use of compound A with alteplase to ischemic stroke than by conventionally known combined use of NBQX and alteplase.

### • Effect in the Known Combinations

On the other hand, in the same models as in the experimental method of the present invention, an effect by the combined use of alteplase with NBQX which is an AMPA receptor antagonist was known (12, 13).
1) Effect for Improving the Neurological Symptoms
   With regard to the effect of reducing the infarct volume by a combined use of NBQX with alteplase, when 90 mg/rat of NBQX (after injection of the blood clot, each 30 mg was administered for three times of 90, 120 and 150 minutes therefrom) was administered 90 minutes after injection of the blood clot and then 20 mg/kg of alteplase was administered during 45 minutes 30 minutes after NBQX administration therefrom (i.e., 120 minutes after the injection of the blood clot), the cerebral infarct volume was statistically significantly suppressed as compared with the control whereby the neurological symptom was improved.
   However, with regard to the improving effect for neurological symptom to a group where alteplase was administered solely, a statistically significant improvement was noted in the present invention while, in a group where NBQX was used jointly, the effect was similar or even worsened.
2) TTW-Extending Effect
   According to the literatures, the effect to ischemic stroke by administration of an AMPA receptor antagonist was the effect by the combined administration before 30 minutes of administration of alteplase. The result means that the TTW (therapeutic time window) in the group where NBQX was jointly used is substantially 90 minutes in rat models.
   On the other hand, in the present invention, when compound A was administered 120 minutes after injection of the blood clot just as in the case of alteplase, a sole administration of compound A 120 minutes after the blood clot injection showed a tendency of improvement while no statistically significant improvement in neurological symptom was noted. On the contrary, the combined administration statistically significantly improved the neurological symptom as compared with sole administration of each of compound A and alteplase.
   From the result, the effect by the combination administration according to the combination of the present invention has a statistically significant effect of reducing the cerebral infarct volume and improving the neurological symptom as compared with a sole administration of each medicament after 120 minutes of the blood clot injection and, further, in rat models, the TTW by the combination use with compound A but NBQX was able to be extended to the same extent as in the case of sole administration of alteplase whereby the combination use with compound A and alteplase is clinically useful effects.

### Example 2. Effect by Combined Use of Pamiteplase and compound A

### <Experimental Methods>

1) Preparation of Cerebral Embolism Models
   Male spontaneously hypertensive rats (SHR) were used. Anesthetization was carried out with 1.0% halothane. During the experiment, rectal temperature of the animals was kept at about 37°C using a heating pad. The cerebral embolism model was prepared by a method (*J. Cerebr. Blood Flow* & *Metab.,* 20:452-457 (2000)) which is a partial modification of a method by Zhang, et al. *(Brain Res.,* 766: 83-92 (1997)). Arterial blood was collected from the femoral artery of a rat for the blood collection into a PE50 tube in which the blood was filled in a length of 50 cm. The PE50 tube filled with the blood was allowed to stand for 2 hours at room temperature and for 22 hours at 4°C to form a blood clot. The PE50 tube filled with the blood clot was cut into pieces of 2.5 cm-length and each piece was connected to a syringe for injection containing a physiological saline using a 23-gauge needle for injection. The blood clot was transferred to a dish containing the physiological saline, washed and transferred into a treated PE50 tube containing a physiological saline. The neck of another rat was incised to ligate the external carotid artery, occipital artery and pterygopaltine artery. A surgical clip was applied to the common and internal carotid arteries to cut the external carotid artery. The top of the treated PE50 tube containing 2.5 cm of the blood clot was inserted into the distal internal carotid artery. The blood clot was then infused and the tube was pulled out after 5 minutes.
   The animals were grouped into the following three. Group A (n = 10): physiological saline-administered control group. Group B (n = 19): a group administered with 1 mg/kg of pamiteplase; pamiteplase was dissolved in a physiological saline (1 mg/mL) and subjected to an intravenous bolus administration over 3 minutes or more. Group C (n = 10): a group administered with 20 mg/kg/hour of compound A and 1 mg/kg of pamiteplase together; compound A was dissolved in a physiological saline and subjected to an intravenous continuous administration for 4 hours while pamiteplase was dissolved in a physiological saline in 1 mg/mL and subjected to an intravenous bolus administration over 3 minutes or more. The medicament was intravenously administered 1 hour after the blood clot injection. Evaluation of cerebral embolism formation by injection of the blood clot was carried out by measuring the cerebral blood flow amount using a laser Doppler flowmetry (Perimed AB, Sweden). The probe for the blood flow measurement was placed on the top of forehead.

### <Items for Evaluation>

1) Infarct Volume
   The animals were sacrificed about 24 hours after injection of the blood clot and the brain was excised and confirmed whether hemorrhagic change was available under a microscope for operation. The excised brain was sliced in ten sections at 2 mm intervals and stained by dipping for 15 minutes in a 2% 2,3,5-triphenyltetrazolium chloride (TCC) solution. The TCC-stained slices were subjected to a digital imaging and a standard computer-assist image analysis system to calculate the infarct volume.
2) Neurological Symptoms Neurological symptoms were observed after 24 hours of the injection of the blood clot. Scoring of the neurological symptoms was carried out according to a method of Bederson et al. *(Stroke,* 17: 472-476 (1986)). Criteria for the score are as follows. Thus, score 0: normal and no worsening of the symptom; score: 1: when the animal was taken up by clipping the tail, forelimb was flexed; score 2: weak resistance to the pushing force from the side and forelimb flexion noted while no circling observed; and score 3: the same symptom as in 2 and the circling were noted.
3) Statistical Means
   Result of the infarct volume was given by a mean value ± standard error while result of the neurological symptom was given by a median value. With regard to the statistical test of the result of the infarct volume, the evaluation was done by carrying out a t-test. On the other hand, with regard to the statistical test of the result of neurological symptoms, the evaluation was done by carrying out a Wilcoxon test. In any of the tests, the value where p < 0.05 was defined to be statistically significant.
4) Reagent
   With regard to 2,3,5-triphenyltetrazolium chloride (Sigma, MO), the commercially available one was purchased.

### <Results>

1) Infarct Volume
   As a result of injection of the blood clot, the cerebral blood flow decreased to about 10% or less than the value prior to the injection and cerebral embolism was formed. The medicament was administered 1 hour after the injection of the blood clot.
   Result of the infarct volume is shown in Fig. 3. The infarct volume of a control group (a group to which a physiological saline was administered) was 360.5 ± 19.3 mm³ (mean value ± standard error). Pamiteplase (1 mg/kg) reduced the infarct volume to 266.2 ± 24.7 mm³. When compound A (20 mg/kg/hour for 4 hours) and pamiteplase (10 mg/kg) were jointly used, the infarct volume became 182.3 ± 25.0 mm³ and the said volume was smaller than that for the pamiteplase-administered group.
2) Neurological Symptoms
   Result of the neurological symptoms is shown in Fig. 4. The neurological symptom in the control group worsened to a score of 3.0 (median value), while that in pamiteplase-administered group gave a score of 2.5 and pamiteplase did not affect the worsened neurological symptom. When compound A and alteplase were jointly used, the neurological symptom gave a score of 2.0 but that did not affect the worsened neurological symptom as compared with the alteplase administration.
   Unexpectedly, sole administration of pamiteplase statistically significantly reduced the infarct volume to the ischemic stroke using spontaneously hypertensive rats.
   When pamiteplase and compound A were jointly administered, infarct volume was statistically significantly reduced as compared with the sole administration of pamiteplase.
   From the above, it was confirmed that, when compound A and a tissue plasminogen activator were jointly used to ischemic stroke accompanied by hypertension, better effect than the conventionally known effect by the combined use of NBQX and alteplase or by a sole administration of a tissue plasminogen activator was available.

### Example 3.

Clinical effect of the present invention was confirmed by the following methods.

### <Test Methods>

Subjects were patients meeting with the following requirements were used as objects and a double blind test was carried out for a group administered with alteplase and a group administered with placebo and also for a group administered with alteplase and a group administered with compound A.
1) Subject Patients
   - Males and females of 18 or more years age
   - Patients to whom alteplase was administered within 3 hours from the onset of ischemic stroke
   - Patients where NIHSS (National Institute of Health stroke scale) was 7-23 and the level of consciousness was 0 or 1
      The patients were further classified into a group where the NIHSS score was 7-14 and another group where it was 15-23.
   - Systolic blood pressure was 185 mmHg or lower while diastolic blood pressure was 110 mmHg or lower
2) Medicaments and Methods of Administration
   With regard to the medicaments to be administered, the subject patients were divided into a group of 7-14 and another group of 15-23 in terms of the NIHSS scores and then randomly assigned by a randomizing desk so that the ratio of (administered with alteplase and compound A) : (administered with alteplase and placebo) is made 1:1.
   With regard to alteplase, the commercially available one was used and 10% of 0.9 mg/kg (upper limit: 90 m) thereof were subjected to an intravenous bolus administration for 1 minute while the remainder was subjected to a continuous intravenous administration for 1 hour.
   With regard to compound A, 1.0 mg/kg/hour was subjected to a continuous intravenous administration for 6 hours and then 0.5 mg/kg/hour was subjected to a continuous intravenous infusion for 18 hours.
   Transfusion bags for compound A and for placebo were covered with a light-shielding cover and administration was carried out where a drip infusion tube was shielded from light as well. With regard to the administration, the initial infusion was carried out at the flow rate of 60 mL/hour for the initial 6 hours and, after the said 6 hours, a sustaining infusion was carried out at the flow rate of 30 mL/hour for 18 hours.
3) Items Measured
   (1) Upon Selection of Subject Patients
      Computed tomography (CT), physical and neurological tests, neurological check, sedation level, NIHSS, 12-lead electrocardiogram, telemetry of electrocardiogram, pulse oxygen measurement, vital sign, body weight, background of the patient, test on pregnancy, measurement of medicament in urine, hematological test, coagulation panel, general clinical test, urinary test
   (2) From Administration of Medicament until 48 Hours Thereafter
      i) at all times
         Pulse oxygen measurement, monitoring of harmful phenomena, therapy by combined medicaments, telemetry of electrocardiogram
      ii) every 1-2 hour(s)
         Vital sign, neurological check (evaluation of consciousness level, pupillary symmetry and optical reaction, ataxia, symptom such as clonic muscle convulsion), sedation level
      iii) 12, 24 and 48 hours after administration
         Abbreviated physical examination, 12-lead electrocardiogram
      iv) 24 and 48 hours after administration
         General clinical test, hematological test, blood coagulation, serum concentration of compound A, urinary test, NIHSS
      v) 24 hours after administration
         CT scan
   (3) Fifth Day after Administration
      CT scan, blood coagulation
   (4) Fifth and 28th Days after Administration
      12-Lead electrocardiogram, hematological test, general clinical test, urinary test
   (5) Fifth, 28th and 90th Days after Administration
      Physical and neurological tests, NIHSS, vital sign, therapy by combination of medicaments, harmful phenomena, Barthel index, modified Rankin scale, Glasgow outcome scale

### <Method of Evaluation>

When the test for each 150 cases of the medium grade group (NIHSS: 7-14) and the severe grade group; (NIHSS: 15-23) was finished, an intermediate analysis was carried out and, finally, for 600 cases where each group comprised 300 cases, the following evaluations were carried out.
i) Primary Evaluation
   Judgment was conducted according to the state of ischemic stroke and to severity scale 90 days after administration of the medicament.
   With regard to the severity scale, the following evaluating items were used.
   (a) NIHSS (NIH Stroke Scale): Judgment is carried out according to the scales of 0-9 (which varies depending upon the item) for level of consciousness, LOC questions, LOC commands, extraocular muscle motion (gaze palsy), hemianopsia, facial palsy, motion of upper limb, motion of lower limb, motion disorder of four limbs, loss of sensation, aphasia, alalia, neglect and, as an additional item, distal motor function (bending of fingers).
   (b) Barthel index: Judgment is carried out according to the scales of 00-15 (which varies depending upon the item) whether or not self-control is possible or nurse care is needed for motion ability in daily life (feeding, moving from wheelchair to bed and return, personal toilet (face washing, hair dressing, tooth brushing, shaving), toileting, bathing, walking on level surface, ascending and descending the stairs, dressing and undressing, continence of bowels, controlling bladder, etc.).
   (c) Modified Rankin scale: Evaluation is carried out according to grades 0-5 whether help or nurse care is necessary for carrying out the motion such as walking for checking the degree of symptom and disturbance.
   (d) Glasgow outcome scale: Evaluation for degree of recovery of general function. Evaluation was done according to the grades 1-5.
      1 = good recovery
      2 = moderate disability (disabled but independent)
      3 = severe disability (conscious but disabled)
      4 = persistent vegetative state
      5 = death
ii) Secondary evaluation
   - On the 28th day after administration of the medicament, degrees of neurological function and disability are evaluated in four scales (modified Rankin scale, Barthel index, NIHSS and Glasgow scale) as the statistic general result.
   - On the 28th and 90th days after the administration, category analysis by the four scales is carried out as the statistic general result. (good outcome of functions: modified Rankin scale is 0 or 1; Barthel index is 95 or more; Glasgow score is 1 [good recovery]; and NIHSS score is 0 or 1)
   - Good outcome of functions on the 5th, 28th and 90th days after the administration (modified Rankin scale is 2 or less and Barthel index is 90 or more).
   - Neurological functions and disturbance in ability on the 5th, 28th and 90th days after the administration is judged by the four scales (NIHSS, modified Rankin scale, Barthel index and Glasgow outcome scale).
   - Changes in the initial NIHS score on the 24th and 48th hours and the fifth, 28th and 90th days after the administration
   - Ratio of the case where NIHSS score is 0 or 1 on the 28th and 90th days after the administration
   - Ratio of the low efficacy on the 90th day after the administration (persistent vegetative state or death in Glasgow outcome scale).

### Industrial Applicability

In the present invention, a combination use of a tissue plasminogen activator and zonampanel or a salt etc. thereof showed better effect for reducing the infarct volume than the case where each of them was solely administered in various cerebral infarction models.

Therefore, a combination in accordance with the present invention is useful for the therapy of ischemic stroke.

While the invention has been described in detail and with reference to specific embodiments thereof, it will be apparent to one skilled in the art that various changes and modifications can be made therein without departing from the scope thereof.

This application is based on U.S. patent applications No. 60/334,556 filed December 3, 2001 and No. 60/361,724 filed March 6, 2002, the entire contents thereof being hereby incorporated by reference.

### Reference Literatures

1. Kohara A., Okada M., Tsutsumi R., Ohno K., Takahashi M., Shimizu-Sasamata M., Shishikura J., Inami H., Sakamoto S. and Yamaguchi T. In-vitro characterization of YM872, a selective, potent and highly water-soluble alpha-amino-3-hydroxy-5-methylisoxazole-4-propionate receptor antagonist. [Journal: Article] *J. Pharm. Pharmacol*. 50:795 - 801 (1998).
2. Kawasaki-Yatsugi S., Ichiki C., Yatsugi S, Takahashi M., Shimizu-Sasamata M., Yamaguchi T. and Minematsu K. Neuroprotective effects of an AMPA receptor antagonist YM872 in a rat transient middle cerebral artery occlusion model. *Neuropharmacol*. 39: 211 - 217 (2000).
3. Takahashi M., Jian Wei Ni., Kawasaki-Yatsugi S., Toya T., Ichiki C., Yatsugi S-I., Koshiya K., Shimizu-Sasamata M. and Yamaguchi T. Neuroprotective efficacy of YM872, an alpha-amino-3-hydroxy-5-methylisoxazole-propionic acid receptor antagonist, after permanent middle cerebral artery occlusion in rats. *J. Pharmacol. Exp. Ther.* 287: 559 - 566 (1998).
4. Kawasaki-Yatsugi S., Yatsugi S., Takahashi M., Toya T., Ichiki C., Shimizu-Sasamata M., Yamaguchi T. and Minematsu K. A novel AMPA receptor antagonist, YM872, reduces infarct size after middle cerebral artery occlusion in rats. *Brain Res.* 793: 39 - 46 (1998).
5. Takahashi M., Jian Wei Ni., Kawasaki-Yatsugi S., Toya T., Yatsugi S-., Shimizu-Sasamata M., Koshiya K., Shishikura J-I., Sakamoto S. and Yamaguchi T. YM872, a novel selective alpha-amino-3-hydroxy-5-methylisoxazole-propionic acid receptor antagonist, reduces brain damage after permanent focal cerebral ischemia in cats. J. *Pharmacol. Exp. Ther.* 284: 467 - 473 (1998).
6. Shimizu-Sasamata M., Kano T., Rogowska J., Wolf GL., Moskowitz MA and Lo EH. YM872, a highly water-soluble AMPA receptor antagonist, preserves the hemodynamic penumbra and reduces brain injury after permanent focal ischemia in rats. Stroke 29: 2141 - 2148 (1998)
7. Haberg A., Takahashi M., Yamaguchi T., Hjelstuen M and Haraldseth O. Neuroprotective effective of the novel glutamate AMPA receptor antagonist YM872 assessed with in vivo MR imaging of rat MCA occlusion. *Brain Res.* 811: 6370 (1998)
8. Kudo M., Aoyama A., Ichimori S. and Fukunaga N. An animal model of cerebral infarction: homologous blood clot emboli in rats. *Stroke* 13: 505 - 508 (1982)
9. Kano T., Katayama Y., Tejima E and Lo EH. Hemorrhagic transformation after fibrinolytic therapy with tissue plasminogen activator in a rat thromboembolic model of stroke. *Brain Res.* 854: 245 - 248 (2000)
10. Meng W., Wang X., Asahi M., Kano T., Asahi K., Ackerman R.H. and Lo E.H. Effects of tissue type plasminogen activator in embolic versus mechanical models of focal cerebral ischemia in rats. *J. Cereb. Blood Flow Metab.* 19: 1316 - 1321 (1999)
11. Bederson J.B., Pitts LH., Tsuji M., Nishimura MC., Davis RL. And Bartkowski H. Rat middle cerebral artery occlusion: evaluation of the model and development of a neurologic examination. *Stroke* 17: 472 - 476 (1986)
12. Overgaard K., Sereghy T., Pedersen H. and Boysen G. Neuroprotection with NBQH and thrombolysis with rt-PA in rat embolic stroke. *Neurol. Res.* 15: 344 - 349 (1993)
13. Meden P., Overgaard K., Sereghy T. and Boysen G. Enhancing the efficacy of thrombolysis by AMPA receptor blockade with NBQX in a rat embolic stroke model. J. *Neurol. Sci.* 119: 209 - 216 (1993)
14. Xue D., Huang ZG., Barnes K., Lesiuk HJ., Smith KE. And Buchan AM. Delayed treatment with AMPA, but not NMDA, antagonists reduces neocortical infarction J. *Cereb. Blood Flow Metab.* 14: 251 - 261 (1994)
15. Graham SH. Chen J., Lan JQ. And Simon RP. A dose-response study of neuroprotection using the AMPA antagonist NBQX in rat focal cerebral ischemia. J. *Pharmacol. Exp. Ther*. 276: 1 - 4 (1996)

## Claims

1. A method for the treatment of ischemic stroke, which comprises administering to a patient a) a therapeutically effective amount of a tissue plasminogen activator as the first component, and (b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component.

2. The method according to claim 1, wherein the second component is administered together with or with a time interval after the administration of the first component.

3. A method for the improvement of function insufficiency accompanied by cerebral infarction, which comprises that a) a therapeutically effective amount of a tissue plasminogen activator as the first component and, (b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component are administered to a patient.

4. The method according to claim 3, wherein the function insufficiency is a neurological symptom.

5. The method according to claims 1 to 3, wherein the first component is a tissue plasminogen activator selected from the group consisting of alteplase and pamiteplase.

6. A combination for the treatment of ischemic stroke, which comprises a) a therapeutically effective amount of a tissue plasminogen activator as the first component and (b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component.

7. A combination for the treatment of ischemic stroke, which comprises a) a therapeutically effective amount of a tissue plasminogen activator as the first component with (b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component and, in the said combination, the said first and second components are intended for different uses in the same or the successive phase or in the apart phases in terms of time.

8. The combination according to claim 6 or 7, wherein the first component is a tissue plasminogen activator selected from the group consisting of alteplase and pamiteplase.

9. The use of a combination of a tissue plasminogen activator with [7-(1H-imidazol-1-yl)-6-nitro-2,3-dioxo-3,4-dihydroquinoxalin-1(2H)-yl]acetic acid or a salt or hydrate thereof for the manufacture of a medicament for the treatment of ischemic stroke.

10. The use according to claim 9, wherein the tissue plasminogen is selected from the group consisting of alteplase and pamiteplase.

11. A pharmaceutical composition for the improvement of function insufficiency accompanied by cerebral infarction, which comprises [7-(1H-imidazol-1-yl)-6-nitro-2,3-dioxo-3,4-dihydroquinoxalin-1(2H)-yl]acetic acid or a salt or hydrate thereof in a therapeutically effective amount when administered to a patient suffered from ischemic stroke and has administered a therapeutically effective amount of a tissue plasminogen activator.

12. A pharmaceutical composition according to claim 11, wherein the composition is begun administering to the patient during the period of a tissue plasminogen activator administration.

13. The use of [7-(1H-imidazol-1-yl)-6-nitro-2,3-dioxo-3,4-dihydroquinoxalin-1(2H)-yl]acetic acid or a salt or hydrate thereof for the manufacture of a medicament for the improvement of function insufficiency accompanied by cerebral infarction, which is administered to the patient to which a therapeutically effective amount of a tissue plasminogen activator has been administered.

14. The use of said compound or a salt or hydrate thereof for the manufacture of a medicament according to claim 13, wherein the medicament is begun administering to the patient during the period of a tissue plasminogen activator administration.

15. A pharmaceutical composition according to claim 11, wherein the composition is begun administering to the patient during the period between an onset of ischemic stroke and four hours after thereof.

16. The use of said compound or a salt thereof for the manufacture of a medicament according to claim 14, wherein the medicament is begun administering to the patient during the period between an onset of ischemic stroke and four hours after thereof.

17. A pharmaceutical composition comprising [7-(1H-imidazol-1-yl)-6-nitro-2,3-dioxo-3,4-dihydroquinoxalin-1(2H)-yl]acetic acid or a salt or hydrate thereof in a therapeutically effective amount for potentiating the reducing effect of cerebral infarction volume by treatment a tissue plasminogen activator to a patient during the period between an onset of ischemic stroke and four hours after thereof.

18. The use of [7-(1H-imidazol-1-yl)-6-nitro-2,3-dioxo-3,4-dihydroquinoxalin-1(2H)-yl]acetic acid or a salt or hydrate thereof for the manufacture of a medicament for potentiating the reducing effect of cerebral infarction volume by treatment a tissue plasminogen activator, wherein the medicament is administered to a patient during the period between an onset of ischemic stroke and four hours after thereof.

19. A method for improving function insufficiency accompanied by cerebral infarction, which comprises administering a therapeutically effective amount of [7-(1H-imidazol-1-yl)-6-nitro-2,3-dioxo-3,4-dihydroquinoxalin-1(2H)-yl]acetic acid or a salt or hydrate thereof to a patient suffered from ischemic stroke and has administered a therapeutically effective amount of a tissue plasminogen activator.

20. The method according to claim 19, wherein administration to the patient starts during the period of a tissue plasminogen activator administration.

21. The method according to claim 19, wherein the administration to the patient starts during the period between an onset of ischemic stroke and four hours after thereof.

22. A method for potentiating the reducing effect of cerebral infarction volume by treatment a tissue plasminogen activator, which comprises administering a therapeutically effective amount of [7-(1H-imidazol-1-yl)-6-nitro-2,3-dioxo-3,4-dihydroquinoxalin-1(2H)-yl]acetic acid or a salt or hydrate thereof to a patient during the period between an onset of ischemic stroke and four hours after thereof.

23. A composition which comprises a) a tissue plasminogen activator as the first component, and b) [7-(1H-imidazol-1-yl)-6-nitro-2,3-dioxo-3,4-dihydroquinoxalin-1(2H)-yl]acetic acid or a salt or hydrate thereof as the second component.

24. The composition according to Claim 23, wherein the first component is a tissue plasminogen activator selected from the group consisting of alteplase and pamiteplase.

25. A composition, which comprises a) a therapeutically effective amount of a tissue plasminogen activator as the first component and b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component, for use as a medicament.

26. A combination which comprises as separate components a) a therapeutically effective amount of a tissue plasminogen activator as the first component with b) a therapeutically effective amount of [7-(1H-imidazol-1-yl)-6-nitro-2,3-dioxo-3,4-dihydroquinoxalin-1(2H)-yl]acetic acid or a salt or hydrate thereof as the second component, for use as a medicament.

27. The use of [7-(1H-imidazol-1-yl)-6-nitro-2,3-dioxo-3,4-dihydroquinoxalin-1(2H)-yl]acetic acid or a salt or hydrate thereof for the preparation of a medicament for improving function insufficiency accompanied by cerebral infarction by administering a therapeutically effective amount of the medicament to a patient suffered from ischemic stroke and has been administered a therapeutically effective amount of a tissue plasminogen activator.

28. The use according to claim 27, wherein the administration starts during the period of the tissue plasminogen activator administration.

29. The use according to claim 27, wherein the administration to the patient starts during the period between the onset of ischemic stroke and four hours after thereof.

30. The use of [7-(1H-imidazol-1-yl)-6-nitro-2,3-dioxo-3,4-dihydroquinoxalin-1(2H)-yl]acetic acid or a salt or hydrate thereof for the preparation of a medicament for potentiating the reducing effect of cerebral infarction volume by treatment with a tissue plasminogen activator by administering a therapeutically effective amount of the medicament to a patient during the period between the onset of ischemic stroke and four hours after thereof.
